# EUROPEAN PATENT APPLICATION

(11) **EP 4 368 242 A1**
(43) Date of publication of application: **15.05.2024**
(21) Application number: 23203107.0
(22) Date of filing: 12.10.2023
(51) Int. Cl.: A61N 1/36

(54) **SYSTEMS AND METHODS FOR GENERATING AND USING STIMULATION PROGRAMS WITH TEMPORAL VARIATION**

(30) Priority: 14.11.2022 US 202263425149 P
(71) Applicant: Boston Scientific Neuromodulation Corporation, Valencia, CA 91355 (US)
(72) Inventor: Juarez Paz, Leon Mauricio, Berlin (DE)
(74) Representative: Vossius & Partner Patentanwälte Rechtsanwälte mbB

(57) **Abstract**

A method for generating a stimulation program includes selecting sets of stimulation parameter values, where at least two stimulation parameter values of each set are selected based on at least one specified clinical effect observed for previous stimulation instances. The at least one specified clinical effect is different for at least two of the sets of stimulation parameter values. The method also includes generating a stimulation program that includes application of stimulation during non-overlapping time intervals, wherein, for each of the non-overlapping time intervals, one of the sets of stimulation parameter values is selected for the application of the stimulation and the selection is different from the selection for an immediately preceding one of the non-overlapping time intervals. The generated stimulation programs can be used for electrical stimulation of tissue.

## Description

### CROSS-REFERENCE TO RELATED APPLICATIONS

This application claims the benefit under 35 U.S. C. § 119(e) of U.S. Provisional Patent Application Serial No. 63/425,149, filed November 14, 2022, which is incorporated herein by reference.

### FIELD

The present invention is directed to the area of implantable electrical stimulation systems and methods of making and using the systems. The present invention is also directed to systems and methods for generating and using temporally-varied stimulation programs that utilize multiple sets of stimulation parameter values.

### BACKGROUND

Implantable electrical stimulation systems have proven therapeutic in a variety of diseases and disorders. For example, spinal cord stimulation systems have been used as a therapeutic modality for the treatment of chronic pain syndromes. Peripheral nerve stimulation has been used to treat chronic pain syndrome and incontinence, with a number of other applications under investigation. Functional electrical stimulation systems have been applied to restore some functionality to paralyzed extremities in spinal cord injury patients. Stimulation of the brain, such as deep brain stimulation, can be used to treat a variety of diseases or disorders.

Stimulators have been developed to provide therapy for a variety of treatments. A stimulator can include a control module (with a pulse generator), at least one lead, and an array of stimulator electrodes on each lead. The stimulator electrodes are in contact with or near the nerves, muscles, brain structures, or other tissue to be stimulated. The pulse generator in the control module generates electrical pulses that are delivered by the electrodes to body tissue.

### BRIEF SUMMARY

One aspect of the invention is a method for generating a stimulation program. The method includes selecting at least two sets of stimulation parameter values, wherein at least two stimulation parameter values of each of the at least two sets of stimulation parameter values are selected based on at least one specified clinical effect observed for each of a plurality of previous stimulation instances, wherein each of the at least one specified clinical effect is a therapeutic effect, a therapeutic response, a side effect, or a testing result and the at least one specified clinical effect is different for at least two of the at least two sets of stimulation parameter values. The method also includes generating a stimulation program that includes application of stimulation during a plurality of non-overlapping time intervals, wherein, for each of the non-overlapping time intervals, one of the at least two sets of stimulation parameter values is selected for the application of the stimulation and the selection is different from the selection for an immediately preceding one of the non-overlapping time intervals.

Another aspect of the invention is a method for electrical stimulation of tissue. The method includes providing a control module and an electrical stimulation lead coupled to the control module, the electrical stimulation lead including a plurality of electrodes and executing a stimulation program on the control module to provide electrode stimulation through the electrodes of the electrical stimulation lead, wherein the stimulation program includes application of stimulation during non-overlapping time intervals, wherein, for each of the non-overlapping time intervals, one of the at least two sets of stimulation parameter values is selected for the application of the stimulation and the selection is different from the selection for an immediately preceding one of the non-overlapping time intervals, wherein at least two of the stimulation parameter values for each of the sets of stimulation parameter values are selected based on at least one specified clinical effect observed for each of a plurality of previous stimulation instances, wherein each of the at least one specified clinical effect is a therapeutic effect, a therapeutic response, a side effect, or a testing result and the at least one specified clinical effect is different for at least two of the at least two sets of stimulation parameter values.

Yet another aspect is a system for generating a stimulation program. The system includes a memory for storing instructions and a processor configured for executing the instructions, wherein the instructions include selecting at least two sets of stimulation parameter values, wherein at least two stimulation parameter values of each of the at least two sets of stimulation parameter values are selected based on at least one specified clinical effect observed for each of a plurality of previous stimulation instances, wherein each of the at least one specified clinical effect is a therapeutic effect, a therapeutic response, a side effect, or a testing result and the at least one specified clinical effect is different for at least two of the at least two sets of stimulation parameter values. The method also includes generating a stimulation program that includes application of stimulation during a plurality of non-overlapping time intervals, wherein, for each of the non-overlapping time intervals, one of the at least two sets of stimulation parameter values is selected for the application of the stimulation and the selection is different from the selection for an immediately preceding one of the non-overlapping time intervals.

A further aspect is a system for electrical stimulation of tissue. The system includes an electrical stimulation lead coupled to the control module, the electrical stimulation lead including a plurality of electrodes; and a control module comprising a memory for storing instructions and a processor configured for executing the instructions, wherein the instructions include executing a stimulation program on the control module to provide electrode stimulation through the electrodes of the electrical stimulation lead, wherein the stimulation program includes application of stimulation during non-overlapping time intervals, wherein, for each of the non-overlapping time intervals, one of the at least two sets of stimulation parameter values is selected for the application of the stimulation and the selection is different from the selection for an immediately preceding one of the non-overlapping time intervals, wherein at least two of the stimulation parameter values for each of the sets of stimulation parameter values are selected based on at least one specified clinical effect observed for each of a plurality of previous stimulation instances, wherein each of the at least one specified clinical effect is a therapeutic effect, a therapeutic response, a side effect, or a testing result and the at least one specified clinical effect is different for at least two of the at least two sets of stimulation parameter values. In at least some embodiments, the system includes a control module and an electrical stimulation lead coupled to the control module, the electrical stimulation lead including a plurality of electrodes.

Another aspect is a non-transitory computer-readable medium having instructions stored thereon for execution by a processor, wherein the instructions include selecting at least two sets of stimulation parameter values, wherein at least two stimulation parameter values of each of the at least two sets of stimulation parameter values are selected based on at least one specified clinical effect observed for each of a plurality of previous stimulation instances, wherein each of the at least one specified clinical effect is a therapeutic effect, a therapeutic response, a side effect, or a testing result and the at least one specified clinical effect is different for at least two of the at least two sets of stimulation parameter values. The method also includes generating a stimulation program that includes application of stimulation during a plurality of non-overlapping time intervals, wherein, for each of the non-overlapping time intervals, one of the at least two sets of stimulation parameter values is selected for the application of the stimulation and the selection is different from the selection for an immediately preceding one of the non-overlapping time intervals.

Yet another aspect is a non-transitory computer-readable medium having instructions stored thereon for execution by a processor, wherein the instructions include executing a stimulation program on the control module to provide electrode stimulation through the electrodes of the electrical stimulation lead, wherein the stimulation program includes application of stimulation during non-overlapping time intervals, wherein, for each of the non-overlapping time intervals, one of the at least two sets of stimulation parameter values is selected for the application of the stimulation and the selection is different from the selection for an immediately preceding one of the non-overlapping time intervals, wherein at least two of the stimulation parameter values for each of the sets of stimulation parameter values are selected based on at least one specified clinical effect observed for each of a plurality of previous stimulation instances, wherein each of the at least one specified clinical effect is a therapeutic effect, a therapeutic response, a side effect, or a testing result and the at least one specified clinical effect is different for at least two of the at least two sets of stimulation parameter values. In at least some embodiments, the system includes a control module and an electrical stimulation lead coupled to the control module, the electrical stimulation lead including a plurality of electrodes.

In at least some aspects, the at least two sets of stimulation parameter values is at least three sets of stimulation parameter values and the stimulation program includes application of stimulation by each of the at least three sets of stimulation parameter values in a repeating sequential order. In at least some aspects, the method further includes modifying an order of the repeating sequential order.

In at least some aspects, the at least two sets of stimulation parameter values is at least three sets of stimulation parameter values and the stimulation program includes application of stimulation by each of the at least three sets of stimulation parameter values in an order that does not repeat a same sequence of at least three time intervals.

In at least some aspects, the at least two sets of stimulation parameter values is at least three sets of stimulation parameter values and the stimulation program includes application of stimulation by each of the at least three sets of stimulation parameter values in a random or semi-random order, wherein randomness of the semi-random order is limited by at least one rule. In at least some aspects, the sets of stimulation parameter values include stimulation parameter values for at least two of the following stimulation parameters: electrode selection, electrode fractionization, stimulation amplitude, pulse width, or pulse frequency, wherein electrode selection includes selection of at least one electrode for delivery of the stimulation and electrode fractionization includes a quantified distribution arrangement of the stimulation among two or more selected electrodes.

In at least some aspects, at least two of the at least two sets of stimulation parameter values differ in electrode selection. In at least some aspects, at least two of the at least two sets of stimulation parameter values differ in stimulation amplitude. In at least some aspects, each of the non-overlapping time intervals has a same duration. In at least some aspects, at least two of the non-overlapping time intervals differ in duration.

Yet another aspect is a system for electrical stimulation of tissue. The system includes a method for storing instructions and a processor configured for executing the instructions, wherein the instructions include any of the methods described above. In at least some embodiments, the system includes a control module and an electrical stimulation lead coupled to the control module, the electrical stimulation lead including a plurality of electrodes.

### BRIEF DESCRIPTION OF THE DRAWINGS

Non-limiting and non-exhaustive embodiments of the present invention are described with reference to the following drawings. In the drawings, like reference numerals refer to like parts throughout the various figures unless otherwise specified.

For a better understanding of the present invention, reference will be made to the following Detailed Description, which is to be read in association with the accompanying drawings, wherein:
FIG. 1 is a schematic view of one embodiment of an electrical stimulation system;
FIG. 2 is a schematic side view of one embodiment of an electrical stimulation lead;
FIG. 3 is a schematic block diagram of one embodiment of a system for generating an electrical stimulation program or for electrical stimulation of tissue;
FIG. 4A illustrates one embodiment of a clinical effects map for postural tremor aligned along a longitudinal axis of the lead;
FIG. 4B illustrates one embodiment of a clinical effects map for postural tremor aligned along a transverse axis of the lead;
FIG. 5A illustrates one embodiment of a clinical effects map for finger tapping speed aligned along a longitudinal axis of the lead;
FIG. 5B illustrates one embodiment of a clinical effects map for finger tapping speed aligned along a transverse axis of the lead;
FIG. 6A illustrates one embodiment of a clinical effects map for upper extremity rigidity aligned along a longitudinal axis of the lead;
FIG. 6B illustrates one embodiment of a clinical effects map for upper extremity rigidity aligned along a transverse axis of the lead;
FIG. 7A illustrates one embodiment of an electrical stimulation program illustrating the pulses and the electrode selection and fractionalization for different time periods of the electrical stimulation program;
FIG. 7B illustrates another embodiment of an electrical stimulation program illustrating the pulses and the electrode selection and fractionalization for different time periods of the electrical stimulation program;
FIG. 8 illustrates yet another embodiment of an electrical stimulation program illustrating the pulses and the electrode selection and fractionalization for different time periods of the electrical stimulation program;
FIG. 9 illustrates one embodiment of an interface for modifying a stimulation program;
FIG. 10 is a flowchart of one embodiment of a method for generating a stimulation program; and
FIG. 11 is a flowchart of one embodiment for electrical stimulation of tissue.

### DETAILED DESCRIPTION

The present invention is directed to the area of implantable electrical stimulation systems and methods of making and using the systems. The present invention is also directed to systems and methods for generating and using temporally-varied stimulation programs that utilize multiple sets of stimulation parameter values.

Suitable implantable electrical stimulation systems include, but are not limited to, a least one lead with at least one electrode disposed on a distal end portion of the lead and at least one terminal disposed on at least one proximal end portion of the lead. Leads include, for example, percutaneous leads, paddle leads, cuff leads, or any other arrangement of electrodes on a lead. Examples of electrical stimulation systems with leads are found in, for example, U.S. Patents Nos. 6,181,969; 6,516,227; 6,609,029; 6,609,032; 6,741,892; 7,244,150; 7,450,997; 7,672,734;7,761,165; 7,783,359; 7,792,590; 7,809,446; 7,949,395; 7,974,706; 8,175,710; 8,224,450; 8,271,094; 8,295,944; 8,364,278; 8,391,985; and 8,688,235; and U.S. Patent Applications Publication Nos. 2007/0150036; 2009/0187222; 2009/0276021; 2010/0076535; 2010/0268298; 2011/0005069; 2011/0004267; 2011/0078900; 2011/0130817; 2011/0130818; 2011/0238129; 2011/0313500; 2012/0016378; 2012/0046710; 2012/0071949; 2012/0165911; 2012/0197375; 2012/0203316; 2012/0203320; 2012/0203321; 2012/0316615; 2013/0105071; and 2013/0197602, all of which are incorporated by reference. In the discussion below, a percutaneous lead will be exemplified, but it will be understood that the methods and systems described herein are also applicable to paddle leads, cuff leads, and other leads.

A lead for electrical stimulation (for example, deep brain or spinal cord stimulation) includes stimulation electrodes that can be ring electrodes, segmented electrodes that extend only partially around the circumference of the lead, or any other type of electrode, or any combination thereof. The segmented electrodes can be provided in sets of electrodes, with each set having electrodes circumferentially distributed about the lead at a particular longitudinal position or across a particular longitudinal region. For illustrative purposes, the leads are described herein relative to use for deep brain stimulation, but it will be understood that any of the leads can be used for applications other than deep brain stimulation, including spinal cord stimulation, peripheral nerve stimulation, or stimulation of other nerves, muscles, and tissues. In particular, stimulation may stimulate specific targets. Examples of such targets include, but are not limited to, the subthalamic nucleus (STN), the internal segment of the globus pallidus (GPi), the ventral intermediate nucleus of the thalamus, the external segment of the globus pallidus (GPe), or the like. In at least some embodiments, an anatomical structure is defined by its physical structure and a physiological target is defined by its functional attributes. In at least some embodiments, the lead may be positioned at least partially within the target, but in other embodiments, the lead may be near, but not inside, the target. The stimulation of tissue can include, but is not limited to, one or more of activation, inhibition, depression, or other modulation of the stimulated tissue.

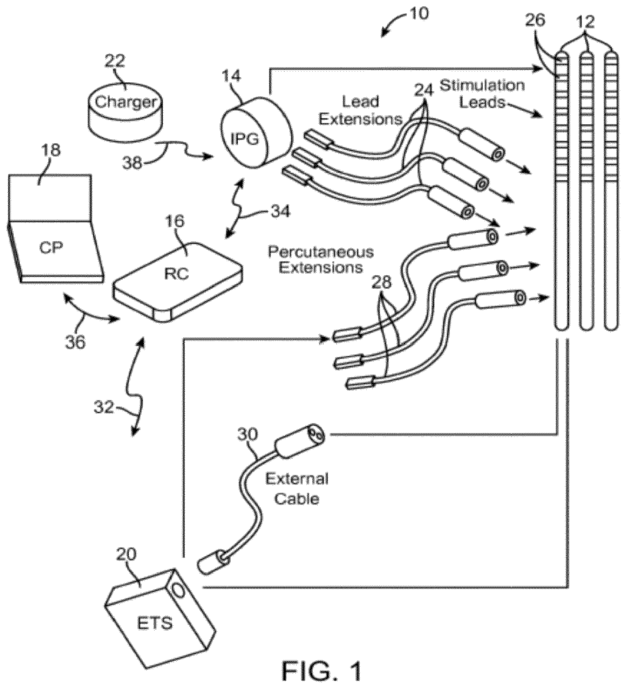

Turning to Figure 1, one embodiment of an electrical stimulation system 10 includes at least one stimulation lead 12 and an implantable pulse generator (IPG) 14. The system 10 can also include at least one of an external remote control (RC) 16, a clinician's programmer (CP) 18, an external trial stimulator (ETS) 20, or an external charger 22.

The IPG 14 is physically connected, optionally via at least one lead extension 24, to the stimulation lead(s) 12. Each lead carries multiple electrodes 26 arranged in an array. The IPG 14 includes pulse generation circuitry that delivers electrical stimulation energy in the form of, for example, a pulsed electrical waveform (i.e., a temporal series of electrical pulses) to the electrode array 26 in accordance with a set of stimulation parameter values. The IPG 14 can be implanted into a patient's body, for example, below the patient's clavicle area or within the patient's buttocks or abdominal cavity. The IPG 14 can have eight stimulation channels which may be independently programmable to control the magnitude of the current stimulus from each channel. In at least some embodiments, the IPG 14 can have more or fewer than eight stimulation channels (for example, 4, 6, 16, 32, or more stimulation channels). The IPG 14 can have one, two, three, four, or more connector ports, for receiving the terminals of the leads.

The ETS 20 may also be physically connected, optionally via the percutaneous lead extensions 28 and external cable 30, to the stimulation leads 12. The ETS 20, which may have similar pulse generation circuitry as the IPG 14, also delivers electrical stimulation energy in the form of, for example, a pulsed electrical waveform to the electrode array 26 in accordance with a set of stimulation parameter values. One difference between the ETS 20 and the IPG 14 is that the ETS 20 is often a non-implantable device that is used on a trial basis after the neurostimulation leads 12 have been implanted and prior to implantation of the IPG 14, to test functioning of the system or the responsiveness of the stimulation that is to be provided. Any functions described herein with respect to the IPG 14 can likewise be performed with respect to the ETS 20.

The RC 16 may be used to telemetrically communicate with or control the IPG 14 or ETS 20 via a uni- or bi-directional wireless communications link 32. Once the IPG 14 and neurostimulation leads 12 are implanted, the RC 16 may be used to telemetrically communicate with or control the IPG 14 via a uni- or bi-directional communications link 34. Such communication or control allows the IPG 14 to be turned on or off and to be programmed with different stimulation parameter sets. The IPG 14 may also be operated to modify the programmed stimulation parameters to actively control the characteristics of the electrical stimulation energy output by the IPG 14. The CP 18 allows a user, such as a clinician, the ability to program stimulation parameters for the IPG 14 and ETS 20 in the operating room and in follow-up sessions.

The CP 18 may perform this function by indirectly communicating with the IPG 14 or ETS 20, through the RC 16, via a wireless communications link 36. Alternatively, the CP 18 may directly communicate with the IPG 14 or ETS 20 via a wireless communications link (not shown). The stimulation parameters provided by the CP 18 are also used to program the RC 16, so that the stimulation parameters can be subsequently modified by operation of the RC 16 in a stand-alone mode (i.e., without the assistance of the CP 18).

For purposes of brevity, the details of the RC 16, CP 18, ETS 20, and external charger 22 will not be further described herein. Details of exemplary embodiments of these devices are disclosed in U.S. Pat. No. 6,895,280, which is expressly incorporated herein by reference. Other examples of electrical stimulation systems can be found at U.S. Patents Nos. 6,181,969; 6,516,227; 6,609,029; 6,609,032; 6,741,892; 7,949,395; 7,244,150; 7,672,734; and 7,761,165; 7,974,706; 8,175,710; 8,224,450; and 8,364,278; and U.S. Patent Application Publication No. 2007/0150036, as well as the other references cited herein, all of which are incorporated by reference.

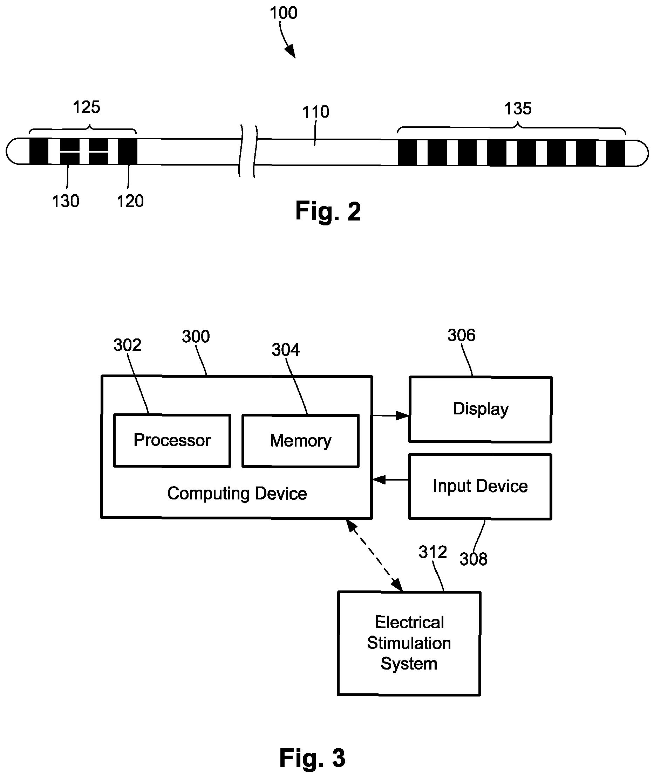

Figure 2 illustrates one embodiment of a lead 100 with electrodes 125 disposed at least partially about a circumference of the lead 100 along a distal end portion of the lead 100 and terminals 135 disposed along a proximal end portion of the lead 100. The lead 100 can be implanted near or within the desired portion of the body to be stimulated such as, for example, the brain, spinal cord, or other body organs or tissues. In one example of operation for deep brain stimulation, access to the desired position in the brain can be accomplished by drilling a hole in the patient's skull or cranium with a cranial drill (commonly referred to as a burr), and coagulating and incising the dura mater, or brain covering. The lead 100 can be inserted into the cranium and brain tissue with the assistance of a stylet (not shown). The lead 100 can be guided to the target location within the brain using, for example, a stereotactic frame and a microdrive motor system. In at least some embodiments, the microdrive motor system can be fully or partially automatic. The microdrive motor system may be configured to perform at least one of the following actions (alone or in combination): insert the lead 100, advance the lead 100, retract the lead 100, or rotate the lead 100.

In at least some embodiments, measurement devices coupled to the muscles or other tissues affected by the target neurons or neural structures, or a unit responsive to the patient or clinician, can be coupled to the IPG 14 or microdrive motor system. The measurement device, user, or clinician can indicate a response by the target muscles or other tissues to the stimulation or recording electrode(s) to further identify the target neurons and facilitate positioning of the stimulation electrode(s). For example, if the target neurons are directed to a muscle experiencing tremors, a measurement device can be used to observe the muscle and indicate changes in, for example, tremor frequency or amplitude in response to stimulation of neurons. Alternatively, the patient or clinician can observe the muscle and provide feedback.

The lead 100 for deep brain stimulation can include stimulation electrodes, recording electrodes, or both. In at least some embodiments, the lead 100 is rotatable so that the stimulation electrodes can be aligned with the target neurons after the neurons have been located using the recording electrodes.

Stimulation electrodes may be disposed on the circumference of the lead 100 to stimulate the target neurons. Stimulation electrodes may be ring shaped so that current projects from each electrode radially from the position of the electrode along a length of the lead 100. In the embodiment of Figure 2, two of the electrodes 125 are ring electrodes 120 . Ring electrodes typically do not enable stimulus current to be directed from only a limited angular range around a lead. Segmented electrodes 130, however, can be used to direct stimulus current to a selected angular range around a lead. When segmented electrodes are used in conjunction with an implantable pulse generator that includes multiple independent current sources, current steering can be achieved to deliver the stimulus more precisely to a position around an axis of a lead (i.e., radial positioning around the axis of a lead). To achieve current steering, segmented electrodes can be utilized in addition to, or as an alternative to, ring electrodes.

The lead 100 includes a lead body 110, terminals 135, at least one ring electrode 120, and at least one set of segmented electrodes 130 (or any other combination of electrodes). The lead body 110 can be formed of a biocompatible, non-conducting material such as, for example, a polymeric material. Suitable polymeric materials include, but are not limited to, silicone, polyurethane, polyurea, polyurethane-urea, polyethylene, or the like. Once implanted in the body, the lead 100 may be in contact with body tissue for extended periods of time. In at least some embodiments, the lead 100 has a cross-sectional diameter of no more than 1.5 mm and may be in the range of 0.5 to 1.5 mm. In at least some embodiments, the lead 100 has a length of at least 10 cm and the length of the lead 100 may be in the range of 10 to 70 cm.

The electrodes 125 can be made using a metal, alloy, conductive oxide, or any other suitable conductive biocompatible material. Examples of suitable materials include, but are not limited to, platinum, platinum iridium alloy, iridium, titanium, tungsten, palladium, palladium rhodium, or the like. Preferably, the electrodes 125 are made of a material that is biocompatible and does not substantially corrode under expected operating conditions in the operating environment for the expected duration of use.

Each of the electrodes 125 can either be used or unused (OFF). When an electrode is used, the electrode can be used as an anode or cathode and carry anodic or cathodic current. In some instances, an electrode might be an anode for a period of time and a cathode for a period of time.

Deep brain stimulation leads may include at least one set of segmented electrodes. Segmented electrodes may provide for superior current steering than ring electrodes because target structures in deep brain stimulation are not typically symmetric about the axis of the distal electrode array. Instead, a target may be located on one side of a plane running through the axis of the lead. Through the use of a radially segmented electrode array ("RSEA"), current steering can be performed not only along a length of the lead but also around a circumference of the lead. This provides precise three-dimensional targeting and delivery of the current stimulus to neural target tissue, while potentially avoiding stimulation of other tissue. Examples of leads with segmented electrodes include U.S. Patents Nos. 8,473,061; 8,571,665; 8,792,993; 9,248,272; 9,775,988; and 10,286,205; U.S. Patent Application Publications Nos. 2010/0268298; 2011/0005069; 2011/0130803; 2011/0130816; 2011/0130817; 2011/0130818; 2011/0078900; 2011/0238129; 2012/0016378; 2012/0046710; 2012/0071949; 2012/0165911; 2012/197375; 2012/0203316; 2012/0203320; 2012/0203321; 2013/0197424; 2013/0197602; 2014/0039587; 2014/0353001; 2014/0358208; 2014/0358209; 2014/0358210; 2015/0045864; 2015/0066120; 2015/0018915; and 2015/0051681, all of which are incorporated herein by reference.

Figure 3 illustrates one embodiment of a system for practicing the invention. The system can include a computing device 300 or any other similar device that includes a processor 302 and a memory 304, a display 306, an input device 308, and, optionally, an electrical stimulation system 312.

The computing device 300 can be a computer, tablet, mobile device, or any other suitable device for processing information. The computing device 300 can be local to the user or can include components that are non-local to the computer including one or both of the processor 302 or memory 304 (or portions thereof). For example, in at least some embodiments, the user may operate a terminal that is connected to a non-local computing device. In other embodiments, the memory can be non-local to the user.

The computing device 300 can utilize any suitable processor 302 including at least one hardware processors that may be local to the user or non-local to the user or other components of the computing device. The processor 302 is configured to execute instructions provided to the processor 302, as described below.

Any suitable memory 304 can be used for the computing device 302. The memory 304 illustrates a type of computer-readable media, namely computer-readable storage media. Computer-readable storage media may include, but is not limited to, nonvolatile, non-transitory, removable, and non-removable media implemented in any method or technology for storage of information, such as computer readable instructions, data structures, program modules, or other data. Examples of computer-readable storage media include RAM, ROM, EEPROM, flash memory, or other memory technology, CD-ROM, digital versatile disks ("DVD") or other optical storage, magnetic cassettes, magnetic tape, magnetic disk storage or other magnetic storage devices, or any other medium which can be used to store the desired information and which can be accessed by a computing device.

Communication methods provide another type of computer readable media; namely communication media. Communication media typically embodies computer-readable instructions, data structures, program modules, or other data in a modulated data signal such as a carrier wave, data signal, or other transport mechanism and include any information delivery media. The terms "modulated data signal," and "carrier-wave signal" includes a signal that has at least one of its characteristics set or changed in such a manner as to encode information, instructions, data, and the like, in the signal. By way of example, communication media includes wired media such as twisted pair, coaxial cable, fiber optics, wave guides, and other wired media and wireless media such as acoustic, RF, infrared, and other wireless media.

The display 306 can be any suitable display device, such as a monitor, screen, display, or the like, and can include a printer. The input device 308 can be, for example, a keyboard, mouse, touch screen, track ball, joystick, voice recognition system, or any combination thereof, or the like.

The electrical stimulation system 312 can include, for example, any of the components illustrated in Figure 1. The electrical stimulation system 312 may communicate with the computing device 300 through a wired or wireless connection or, alternatively or additionally, a user can provide information between the electrical stimulation system 312 and the computing device 300 using a computer-readable medium or by some other mechanism. In at least some embodiments, the computing device 300 may include part of the electrical stimulation system, such as, for example, the IPG 14, CP 18, RC 16, ETS 20, or any combination thereof.

The methods and systems described herein may be embodied in many different forms and should not be construed as limited to the embodiments set forth herein. Accordingly, the methods and systems described herein may take the form of an entirely hardware embodiment, an entirely software embodiment or an embodiment combining software and hardware aspects. Systems referenced herein typically include memory and typically include methods for communication with other devices including mobile devices. Methods of communication can include both wired and wireless (for example, RF, optical, or infrared) communications methods and such methods provide another type of computer readable media; namely communication media. Wired communication can include communication over a twisted pair, coaxial cable, fiber optics, wave guides, or the like, or any combination thereof. Wireless communication can include RF, infrared, acoustic, near field communication, Bluetooth^{™}, or the like, or any combination thereof.

Given the number of electrodes and the possibility of using multiple electrodes together, along with the directionality provided by segmented electrodes, it can be challenging to identify a set(s) of stimulation parameters that provide a desirable level of treatment for a condition or disorder. The number of degrees of programming freedom can be daunting. It is, therefore, useful to identify methods and systems that can facilitate programming and identifying suitable stimulation parameters for stimulation programs.

The selection of settings for stimulation, such as deep brain electrical stimulation, can include the determination of clinical effects (for example, therapeutic effects, therapeutic responses, side effects, or testing results) to different stimulation settings and may include the variation of one or more parameters such as, for example, electrode selection, stimulation amplitude, duration, pulse frequency, pulse duration, or the like or any combination thereof. The clinical effects can be therapeutic effects, therapeutic responses, side effects, testing results (e.g., results of one or more testing protocols), or any combination thereof. It will be understood that the term "clinical effects" and terms "responses" and "clinical responses" are interchangeable herein.

A clinical effects map that displays one or more therapeutic effects, therapeutic responses, side effects, or test results (or any combination thereof) for each of one or more stimulation instances (or estimated clinical effects for stimulation instances) can guide stimulation programming. One example of a two-dimensional clinical effects map is described in U.S. Patent Application Publication No. 2014/0277284, incorporated herein by reference in its entirety. In this particular example, the x axis of the clinical effects map corresponds to stimulation amplitude and the y axis corresponds to the position (or a composite position) of the stimulating electrode(s) along the lead. In this particular example, the y axis provides spatial information regarding the stimulation, but the x axis does not. One example of a three-dimensional clinical effects map is described in U.S. Patent No. 10,071,249, incorporated herein by reference in its entirety. The x and z axes correspond to the electrode selection and the y axis corresponds to the amplitude. Two of the axes provide spatial information but this spatial information corresponds to the position (or a composite position) of one or more stimulating electrodes on the lead. U.S. Provisional Patent Application Serial No. 63/288,153, incorporated herein by reference in its entirety, illustrates other embodiments of a clinical effects map.

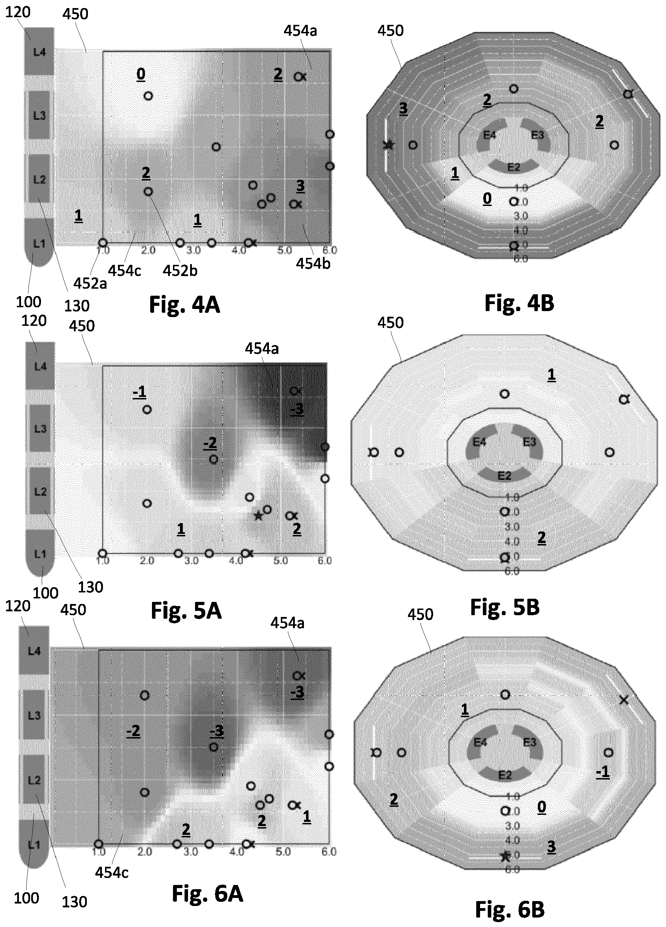

In at least some embodiments, the clinical effects are observed for multiple symptoms or testing protocols. For example, deep brain stimulation may be used to treat motor diseases. Observation of different disease symptoms or testing protocols may be used to measure or determine the clinical effects of the stimulation. Figures 4A, 4B, 5A, 5B, 6A, and 6B illustrate embodiments of different clinical effects maps 450 for postural tremor (Figures 4A and 4B), finger tapping speed (Figures 5A and 5B), and upper extremity rigidity (Figures 6A and 6B).

Figures 4A, 5A, and 6A each illustrate a longitudinal clinical effects map 450 taken along a length of the distal portion of the lead 100 with ring electrodes 120 and segmented electrodes 130 (which are also known as directional electrodes) at longitudinal levels L1, L2, L3, and L4 along the lead. The electrode position is the vertical axis and the stimulation amplitude is the horizontal axis. Positions between the levels can correspond to selection of two or more electrodes instead of a single electrode, as well as variation in the fractionalization of the current or voltage between the selected electrodes.

Figures 4B, 5B, and 6B each illustrate a cross-sectional slice of the clinical effects map 450 taken at L2 with electrodes E1, E2, and E3 at different circumferential positions. The radial coordinate represents stimulation amplitude in Figures 4B, 5B, and 6B. The angular coordinate represent electrode selection and fractionalization (i.e., the distribution of the current or voltage between the selected electrodes) which can include selection of more than one of the electrode.

A clinical effects map is typically generated from multiple stimulation instances. Each stimulation instance is defined by one or more stimulation parameters (for example, electrode selection, stimulation amplitude, pulse width, pulse frequency, or the like) that are used for the stimulation. In Figure 4A, points 452a, 452b, as well as the other points on Figures 4A to 6B, correspond to different stimulation instances. As an example, point 452a corresponds to stimulation using a ring electrode 120 and point 452b corresponds to stimulation using one or more electrodes 130.

In at least some embodiments, the stimulation parameters for the stimulation instances can be manually programmed or the stimulation instances can be a set of stimulations performed using an automated programming sequence or any combination thereof. In at least some embodiments, an automated programming sequence may also utilize the clinical effects from preceding stimulation instances to inform or select the next or succeeding stimulation instances. U.S. Patent No. 10,603,498, incorporated herein by reference in its entirety, describes examples of such automated programming.

For each stimulation instance, one or more clinical effects are observed, measured, or otherwise obtained. The clinical effect(s) can be therapeutic effect(s) or response(s), side effect(s), test results, or the like or any combination thereof. For example, Figures 4A and 4B represent observation of the therapeutic effect on postural tremor, Figures 5A and 5B represent observation of results of a finger tapping test, and Figures 6A and 6B represent observation of the therapeutic effect on upper extremity rigidity.

For each stimulation instance (e.g., each point) presented on Figures 4A, 4B, 5A, 5B, 6A, and 6B, a measure or score for at least one clinical effect is obtained or determined and then recorded. The observation, determination, or input of the clinical effect(s) may be performed by the user, the patient, or any other suitable person or the clinical effect(s) can be observed or determined by a processor of the system or a sensor or other device.

Examples of measurements or scores for clinical effects include, but are not limited to, manually assessed clinical scores, sensor-derived scores or values, electrophysiological signals, or the like or any combination thereof. For example, a user may input a quantitative or qualitative score based on visual observation of the patient, a sensor, or data (for example, an EEG or ECG or the like); verbal feedback from the patient; an evoked compound action potential (ECAP) or an evoked resonant neural activity (ERNA); or the like.

As another example, at least one sensor (for example, a haptic sensor, accelerometer, gyroscope, EEG, EMG, camera, or the like) may be used to observe or determine one or more clinical effects and may provide a quantitative or qualitative measurement, score, or other value (either directly to the processor or through a programmer, a user, the patient, or another person) that represents one of the clinical effects. A quantitative or qualitative value can indicate, for example, at least one characteristic of a symptom (for example, tremor), a therapeutic effect or side effect (for example, change in the patient's balance), electrical activity, or the like. The clinical effect may be indicative of a therapeutic effect or a side effect or both. Moreover, in at least some embodiments, more than one clinical effect can be observed, determined, or input for each stimulation instance.

In at least some embodiments, such as in Figures 4A to 6B, the clinical effects map utilizes coloring, shading, brightness, or any other suitable graphical elements to identify the clinical effects, measurements, scores, or other indications. For example, a clinical effects map can utilize one color (e.g., green) to signify regions where stimulation produces at least one therapeutic effect and another color (e.g., yellow) to signify regions where stimulation produces at least one side effect. In at least some embodiments, for regions that produce both therapeutic and side effects, the coloring (or other graphical elements) may be weighted based on the intensity or strength of the therapeutic effect(s) and side effect(s). In at least some embodiments, the shade or brightness of the color may be used to indicate the strength or intensity of the therapeutic effect(s) or side effect(s).

As another example, a clinical effects map can utilize different colors, shading, brightness, or the like to indicate a measure or score where one color, shade, or brightness value can represent the highest measure or score and other colors, shades, or brightness values can represent other measures or scores. In the original clinical effects maps from which Figures 4A, 4B, 5A, 5B, 6A, and 6B are generated, color and shading was used to represent the measure or score with dark green representing the highest scores, light shades of green representing lower scores, and yellow, orange, and red representing even lower scores. Because these colors are not present in Figures 4A, 4B, 5A, 5B, 6A, and 6B, the Figures have been annotated with underlined numbers, ranging from 0 to 5, to represent the measure or score where 5 is the best result for the clinical effect.

A clinical effects map can facilitate identifying one or more sets of stimulation parameter values that provide a good response and sets of stimulation parameter values that do not provide a good response (or may even provide a poor response). Comparing the clinical effects maps for different clinical effects will often indicate that some sets of stimulation parameter values that are good for producing one clinical effect may provide little or no response for another clinical effect. As an example, in Figures 4A, 5A, and 6A, the upper right quadrant 454a, lower right quadrant 454b, and lower left quadrant 454c show good responses for postural tremor (Figure 4A), but the upper right quadrant 454a shows a poor response for finger tapping speed (Figure 5A) and upper extremity rigidity (Figure 6A) and the lower left quadrant 454c shows a poor response for upper extremity rigidity (Figure 5A).

In many conventional arrangements, a set of stimulation parameter values is selected by balancing these clinical effects. For example, a weighted scoring system may be used to select a set of stimulation parameters based on the scores for the individual clinical effects to two or more symptoms or tests with an optional weighting for each symptom or test. This approach can provide beneficial therapeutic stimulation, but there may be trade-offs in order to balance the therapy.

In contrast, as described herein, two or more sets of stimulation parameter values are selected, with each set of stimulation parameter values selected based on a different clinical effect or set of clinical effects. Stimulation using each set is temporally interleaved with the other set(s) to produce a temporally varied stimulation. For example, three different sets of stimulation parameter values can be determined based on the results for each of postural tremor (Figures 4A and 4B), finger tapping speed (Figures 5A and 5B), and upper extremity rigidity (Figures 6A and 6B), respectively. These three sets of stimulation parameter values can be applied to the patient in a temporally interleaved sequence.

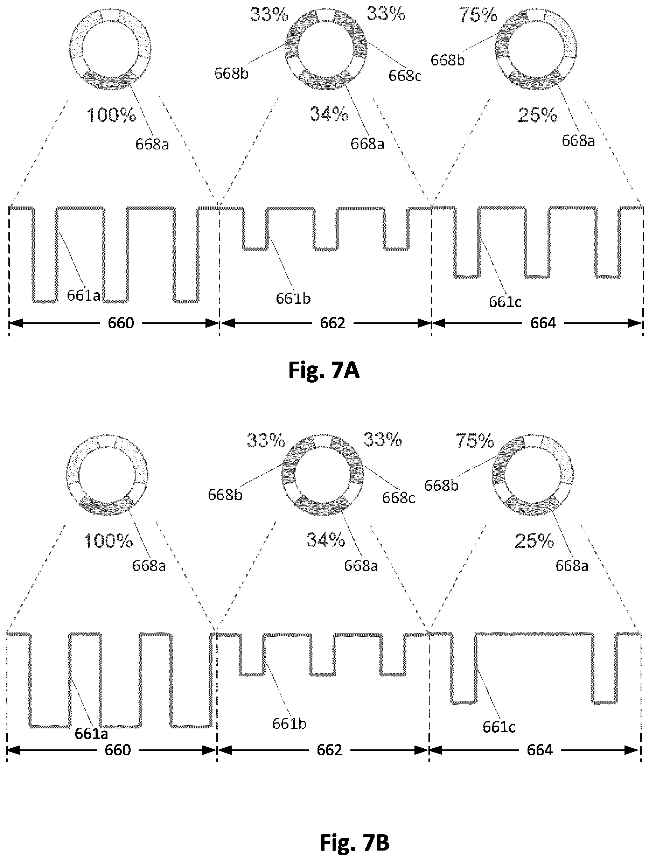

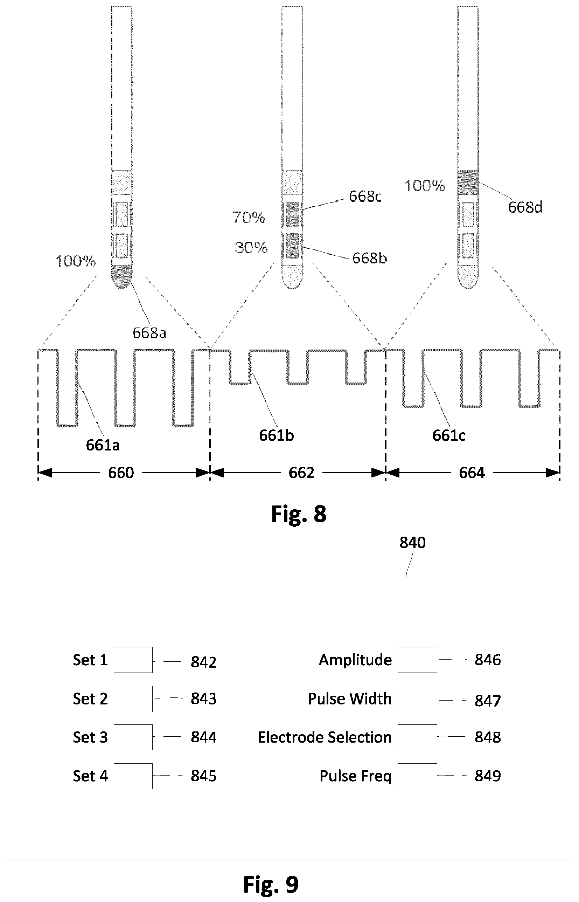

Figures 7A, 7B, and 8 illustrate three different embodiments of three sets of stimulation parameter values that are temporally interleaved. In both embodiments, there is a first time period 660 in which stimulation is produced using a first set of stimulation parameter values, a second time period 662 in which stimulation is produced using a second set of stimulation parameter values, and a third time period 664 in which stimulation is produced using a third set of stimulation parameter values.

In the embodiment illustrated in Figure 7A, electrode selection and amplitude of stimulation is altered to produce the three different sets of stimulation parameter values. For example, in the first time period 660, stimulation pulses 661a are produced using a single electrode 668a with a relatively high stimulation amplitude. In the second time period 662, stimulation pulses 661b are produced using three electrode 668a, 668b, 668c with equal (or nearly equal) amplitude distribution between the three electrodes of a relatively low stimulation amplitude. In the third time period 664, stimulation pulses 661c are produced using two electrodes 668a, 668b with unequal amplitude distribution (25% on electrode 668a and 75% on electrode 668b) at an intermediate stimulation amplitude.

In the embodiment illustrated in Figure 7B, additional stimulation parameters are varied. For example, in the first time period 660 of Figure 7B, the pulse width or pulse duration is longer than in the other two time periods 662, 664. The pulse frequency is lower in the third time period 664 of Figure 7B than in the other two time periods 660, 662.

In the embodiments, illustrated in Figures 7A and 7B, the electrode selection and fractionalization (i.e., distribution of the amplitude) is varied circumferentially among electrodes at the same longitudinal level of the lead. In the embodiment illustrated in Figure 8, the electrode selection and fractionalization is varied longitudinally along the lead. In the first time period 660, stimulation pulses 661a are produced using a single ring electrode 668a with a relatively high stimulation amplitude. In the second time period 662, stimulation pulses 661b are produced using two sets 668b, 668c, of three electrode 668a with different amplitude distribution between the two sets of a relatively low stimulation amplitude. The distribution of the amplitude within the electrodes of each set 668b, 668c can be equal or unequal. In the third time period 664, stimulation pulses 661c are produced using a single ring electrode 668d at an intermediate stimulation amplitude.

It will be understood that any combination of circumferential or longitudinal variation of the electrode selection and fractionalization can be used.

In at least some embodiments, the duration of the stimulation (e.g., time periods 660, 662, 664 of Figure 7A) for each of the sets of stimulation parameter values can be the same. In other embodiments, the duration of the stimulation for each of the sets of stimulation parameter values can be different so that, for example, the first time period may be longer than the second and third time periods. For example, one set can be used to stimulate for a duration that is twice the duration of another set (e.g., time period 660 can be twice the duration of time period 662). In at least some embodiments, the duration of the stimulation for at least one (or even each) of the sets of stimulation parameter values can be varied between instances of that set.

Examples of other stimulation parameters that can be varied include, but are not limited to, electrode selection, stimulation amplitude, distribution of the stimulation amplitude among the electrodes, number of pulses, pulse duration, pulse frequency, pulse pattern, duration of stimulation, or the like or any combination thereof.

In at least some embodiments, stimulation using each set of stimulation parameter values can be delivered using a different channel of the control module. In at least some embodiments, stimulation can be delivered from the same channel using two or more of the sets of stimulation parameter values that are temporally interleaved.

Examples are presented herein for stimulation patterns and orders using a first set of stimulation parameter values denoted by the letter "A", a second set of stimulation parameter values denoted by the letter "B", and a third set of stimulation parameter values denoted by the letter "C". These designations are used herein to provide examples of the temporally interleaved stimulation using different sets of stimulation parameter values.

A repeating sequential order or pattern is one in which one or more base sequences (each base sequence including at least two of the sets of stimulation parameter values) are repeated. In at least some embodiments, the stimulation represented by the three time periods 660, 662, 664 is temporally interleaved in the repeating sequential order of "ABCABC...." In this repeating sequential order, each set of stimulation parameter values is used in the same order prior to a repeat of any of the other sets. In at least some embodiments, such as this one, the sets of stimulation parameter values represented by each of the three time periods 660, 662, 664 is provided to the patient an equal number of times during the overall stimulation period.

In other embodiments, the number of times that the stimulation represented by each of the three time periods 660, 662, 664 is provided to the patient can be unequal. For example, other sequential orders can be used including those in which at least one set of stimulation parameter values is repeated prior to use of another one of the sets of stimulation parameter values. Examples of such repeating sequential orders, which include more instances of "A" and "B" than "C" are "ABCABABCABABCAB..." or "ABABABCABABABC...." Any other suitable repeating sequential order can be used.

In at least some embodiments, a sequential order includes a base sequence that has at least one instance of each of the sets of stimulation parameter values. In the examples above, the base sequences are "ABC", "ABCAB", and "ABABABC". In at least some embodiments, a repeating sequential order can include two or more base sequences, such as "ABCABCABABABCABCABC... " where the base sequences are "ABC" and "ABABABC".

Non-repeating orders can also be used. In at least some embodiments, the stimulations represented by the three time periods 660, 662, 664 are presented in a non-repeating order (e.g., an order with no clear base sequences). Examples of non-repeating orders include, but are not limited to, planned or predetermined non-repeating orders, random (or pseudorandom) orders, semi-random orders (e.g., an otherwise random order in which one or more rules limit the randomness), or any other suitable order. In at least some of these embodiments, rules may be applied to ensure that a certain level of stimulation using each set of stimulation parameter values is achieved. For example, a rule can require that a minimum amount of time or number of stimulations be performed using a particular set over a specified time period, a rule can set a maximum period of time between stimulation using a particular set, a rule can set a maximum number of consecutive uses of a set, or the like or any combination thereof. For example, a rule could require that set "A" be applied at least eight times over a five minute period or a rule could require that each set be applied at least once in every group of 10 or 20 time periods.

In at least some embodiments, an electrical stimulation system can permit a user to select, modify, or adjust the stimulation parameters manually. For example, a user can employ the CP18 or RC 16 to select, modify, or adjust the stimulation parameters. Turning to Figure 9, a user interface may include user-selectable controls for selecting or adjusting one or more stimulation parameters for each set of stimulation parameter values. Figure 9 shows a display 840 with user-selectable controls for selecting a general setting for making selections for each of the sets of stimulation parameter values individually. In the illustrated embodiment, four sets 842-845 are available for selection. In the illustrated embodiment, the user interface includes user-selectable controls 846-849 that enable a user to select values for different stimulation parameters, such as amplitude 846, pulse width 847, electrode selection 848, and pulse frequency 849.

Similarly, in at least some embodiments, an electrical stimulation system can permit a user to select, modify, or adjust an order of the use of the sets of stimulation parameter values. In at least some embodiments, a user interface can allow a user to define, select, or modify an order (for example, a repeating sequential order or a non-sequential order.) In at least some embodiments, the user interface can allow a user to define, select, or modify one or more base sequences for the order. In at least some embodiments, the user interface can allow the user to select a random (or pseudorandom) order, a semi-random order, or other non-repeating order. In at least some embodiments, a user interface can allow selection, definition, or modification of one or more rules that modify or define the order.

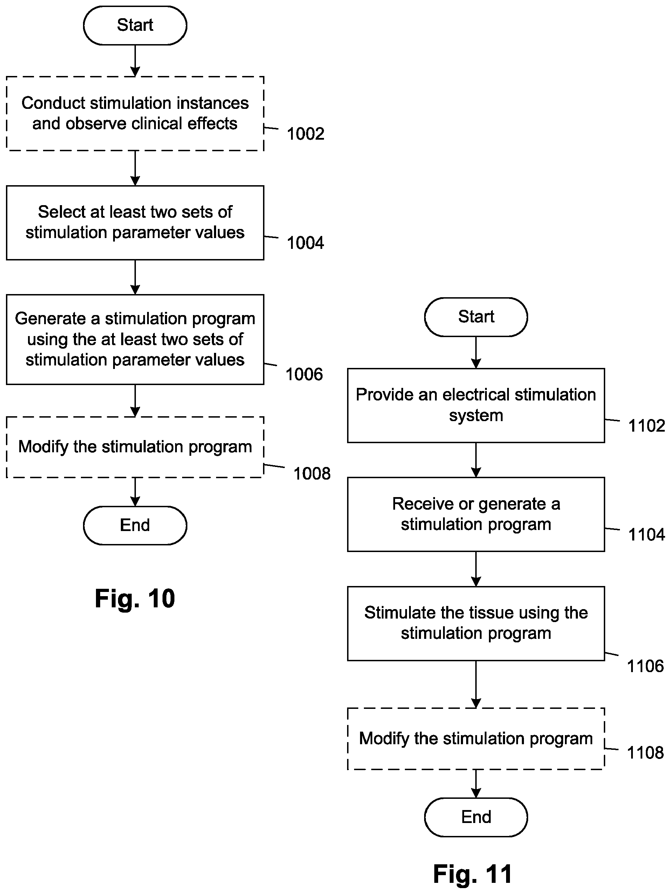

Figure 10 is a flow chart of one embodiment of a method for generating a stimulation program. In optional step 1002, multiple stimulation instances are conducted using different stimulation parameter values and one or more clinical effects are observed for each stimulation instance. Alternatively, the stimulation instances and clinical effects (or stimulation parameter values that are based on the stimulation instances and clinical effects) are provided to the user generating the stimulation program instead of the user performing this step. In at least some embodiments, one or more (or all) of the stimulation instances are performed on the patient for whom the stimulation program is generated. In at least some embodiments, one or more (or all) of the stimulation instances are performed on patients other than the patient for whom the stimulation program is generated.

In step 1004, at least two sets of stimulation parameter values are selected. For each of the sets of stimulation parameter values, at least two of the stimulation parameter values are selected based on at least one specified clinical effect observed for the previous stimulation instances. In at least some embodiments, each of the specified clinical effects can be a therapeutic effect, a therapeutic response, a side effect, or a testing result. In at least some embodiments, each of the specified clinical effects is a therapeutic effect, a therapeutic response, or a side effect. The specified clinical effects differ for each of the at least two sets of stimulation parameter values. Using the examples described above, a different set of stimulation parameter values can be selected for each of the following three clinical effects, based on the observation of these clinical effects in previous stimulation instances: postural tremor, finger tapping speed, and upper extremity rigidity. Any other suitable clinical effect or set of clinical effects can be the basis upon which a set of stimulation parameter values is chosen.

In step 1006, a stimulation program is generated using the sets of stimulation parameter values. The stimulation program includes application of stimulation during multiple non-overlapping time intervals. For each of the non-overlapping time intervals, one of the sets of stimulation parameter values is selected for the application of the stimulation, where that selection is different from the selection for an immediately preceding one of the non-overlapping time intervals.

In optional step 1008, the stimulation program is modified by the user. For example, one of more stimulation parameter values of one or more of the sets of stimulation parameter values can be modified, the order of selection of the sets of stimulation parameter values can be modified, or the like or any combination thereof.

Figure 11 illustrates one embodiment of a method for electrical stimulation of tissue. In step 1102, an electrical stimulation system is provided. The electrical stimulation system can include a control module and an electrical stimulation lead with electrodes, as described above.

In step 1104, the electrical stimulation system receives or generates a stimulation program. The stimulation program includes application of stimulation during multiple non-overlapping time intervals. For each of the non-overlapping time intervals, one of at least two sets of stimulation parameter values is selected for the application of the stimulation, where that selection is different from the selection for an immediately preceding one of the non-overlapping time intervals. For each of the sets of stimulation parameter values, at least two stimulation parameter values of each of the at least two sets of stimulation parameter values are selected based on at least one specified clinical effect observed for previous stimulation instances. In at least some embodiments, each of the specified clinical effects can be a therapeutic effect, a therapeutic response, a side effect, or a testing result. In at least some embodiments, each of the specified clinical effects is a therapeutic effect, a therapeutic response, or a side effect. The specified clinical effects differ for each of the sets of stimulation parameter values.

In step 1106, the tissue is stimulated by the electrical stimulation system according to the stimulation program. In optional step 1108, the stimulation program is modified by the user. For example, one of more stimulation parameter values of the sets of stimulation parameter values can be modified, the order of selection of the sets of stimulation parameter values can be modified, or the like or any combination thereof.

It will be understood that each block of the flowchart illustrations, and combinations of blocks in the flowchart illustrations and methods disclosed herein, can be implemented by computer program instructions. These program instructions may be provided to a processor to produce a machine, such that the instructions, which execute on the processor, create means for implementing the actions specified in the flowchart block or blocks disclosed herein. The computer program instructions may be executed by a processor to cause a series of operational steps to be performed by the processor to produce a computer implemented process. The computer program instructions may also cause at least some of the operational steps to be performed in parallel. Moreover, some of the steps may also be performed across more than one processor, such as might arise in a multi-processor computer system. In addition, at least one process may also be performed concurrently with other processes, or even in a different sequence than illustrated without departing from the scope or spirit of the invention.

The computer program instructions can be stored on any suitable computer-readable medium including, but not limited to, RAM, ROM, EEPROM, flash memory or other memory technology, CD-ROM, digital versatile disks ("DVD") or other optical storage, magnetic cassettes, magnetic tape, magnetic disk storage or other magnetic storage devices, in the cloud or other non-local site, or any other medium which can be used to store the desired information and which can be accessed by a computing device.

A system can include one or more processors that can perform the methods (in whole or in part) described above. In at least some embodiments, some or all of the method may be performed using one or more non-local processor(s) (for example, processors in another device or in the cloud.) The methods, systems, and units described herein may be embodied in many different forms and should not be construed as limited to the embodiments set forth herein. Accordingly, the methods, systems, and units described herein may take the form of an entirely hardware embodiment, an entirely software embodiment or an embodiment combining software and hardware aspects. The methods described herein can be performed using any type of processor or any combination of processors where each processor performs at least part of the process. In at least some embodiments, the processor may include more than one processor.

The above specification provides a description of the structure, manufacture, and use of the invention. Since many embodiments of the invention can be made without departing from the spirit and scope of the invention, the invention also resides in the claims hereinafter appended.

## Claims

1. A method for generating a stimulation program, the method comprising:
selecting at least two sets of stimulation parameter values, wherein at least two stimulation parameter values of each of the at least two sets of stimulation parameter values are selected based on at least one specified clinical effect observed for each of a plurality of previous stimulation instances, wherein each of the at least one specified clinical effect is a therapeutic effect, a therapeutic response, a side effect, or a testing result and the at least one specified clinical effect is different for at least two of the at least two sets of stimulation parameter values; and
generating a stimulation program that comprises application of stimulation during a plurality of non-overlapping time intervals, wherein, for each of the non-overlapping time intervals, one of the at least two sets of stimulation parameter values is selected for the application of the stimulation and the selection is different from the selection for an immediately preceding one of the non-overlapping time intervals.

2. The method of claim 1, wherein at least two of the at least two sets of stimulation parameter values differ in electrode selection.

3. The method of any one of claims 1 or 2, wherein at least two of the at least two sets of stimulation parameter values differ in stimulation amplitude.

4. The method of any one of claims 1 to 3, wherein the sets of stimulation parameter values comprise stimulation parameter values for at least two of the following stimulation parameters: electrode selection, electrode fractionization, stimulation amplitude, pulse width, or pulse frequency, wherein electrode selection comprises selection of at least one electrode for delivery of the stimulation and electrode fractionization comprises a quantified distribution arrangement of the stimulation among two or more selected electrodes.

5. A method for electrical stimulation of tissue, the method comprising:
providing a control module and an electrical stimulation lead coupled to the control module, the electrical stimulation lead comprising a plurality of electrodes;
executing a stimulation program on the control module to provide electrode stimulation through the electrodes of the electrical stimulation lead, wherein the stimulation program comprises application of stimulation during non-overlapping time intervals, wherein, for each of the non-overlapping time intervals, one of the at least two sets of stimulation parameter values is selected for the application of the stimulation and the selection is different from the selection for an immediately preceding one of the non-overlapping time intervals, wherein at least two of the stimulation parameter values for each of the sets of stimulation parameter values are selected based on at least one specified clinical effect observed for each of a plurality of previous stimulation instances, wherein each of the at least one specified clinical effect is a therapeutic effect, a therapeutic response, a side effect, or a testing result and the at least one specified clinical effect is different for at least two of the at least two sets of stimulation parameter values.

6. The method of claim 5, wherein at least two of the at least two sets of stimulation parameter values differ in electrode selection.

7. The method of any one of claims 5 or 6, wherein at least two of the at least two sets of stimulation parameter values differ in stimulation amplitude.

8. The method of any one of claims 5 to 7, wherein the sets of stimulation parameter values comprise stimulation parameter values for at least two of the following stimulation parameters: electrode selection, electrode fractionization, stimulation amplitude, pulse width, or pulse frequency, wherein electrode selection comprises selection of at least one electrode for delivery of the stimulation and electrode fractionization comprises a quantified distribution arrangement of the stimulation among two or more selected electrodes.

9. The method of any one of claims 1 to 8, wherein the at least two sets of stimulation parameter values is at least three sets of stimulation parameter values and the stimulation program comprises application of stimulation by each of the at least three sets of stimulation parameter values in a repeating sequential order.

10. The method of claim 9, further comprising modifying an order of the repeating sequential order.

11. The method of any one of claims 1 to 8, wherein the at least two sets of stimulation parameter values is at least three sets of stimulation parameter values and the stimulation program comprises application of stimulation by each of the at least three sets of stimulation parameter values in an order that does not repeat a same sequence of at least three time intervals.

12. The method of any one of claims 1 to 8, wherein the at least two sets of stimulation parameter values is at least three sets of stimulation parameter values and the stimulation program comprises application of stimulation by each of the at least three sets of stimulation parameter values in a random or semi-random order, wherein randomness of the semi-random order is limited by at least one rule.

13. The method of any one of claims 1 to 12, wherein at least two of the non-overlapping time intervals differ in duration.

14. The method of any one of claims 1 to 12, wherein each of the non-overlapping time intervals has a same duration.

15. A system for generating a stimulation program, comprising:
a memory for storing instructions; and
a processor configured for executing the instructions, wherein the instructions comprise
selecting at least two sets of stimulation parameter values, wherein at least two stimulation parameter values of each of the at least two sets of stimulation parameter values are selected based on at least one specified clinical effect observed for each of a plurality of previous stimulation instances, wherein each of the at least one specified clinical effect is a therapeutic effect, a therapeutic response, a side effect, or a testing result and the at least one specified clinical effect is different for at least two of the at least two sets of stimulation parameter values; and
generating a stimulation program that includes application of stimulation during a plurality of non-overlapping time intervals, wherein, for each of the non-overlapping time intervals, one of the at least two sets of stimulation parameter values is selected for the application of the stimulation and the selection is different from the selection for an immediately preceding one of the non-overlapping time intervals.
